(19) Europäisches Patentamt
European Patent Office
Office européen des brevets

(11) **EP 1 820 849 B1**

(12) **FASCICULE DE BREVET EUROPEEN**

(45) Date de publication et mention
de la délivrance du brevet:
**21.01.2009 Bulletin 2009/04**

(51) Int Cl.:
*C12N 1/20* (2006.01) *A23C 9/123* (2006.01)

(21) Numéro de dépôt: **06290283.8**

(22) Date de dépôt: **20.02.2006**

(54) **Nouvelles souches de Lactobacillus helveticus**

Neue Stämme von Lactobacillus helveticus

New strains of Lactobacillus helveticus

(84) Etats contractants désignés:
**AT BE BG CH CY CZ DE DK EE ES FI FR GB GR HU IE IS IT LI LT LU LV MC NL PL PT RO SE SI SK TR**

(43) Date de publication de la demande:
**22.08.2007 Bulletin 2007/34**

(73) Titulaire: **COMPAGNIE GERVAIS DANONE**
**92300 Levallois Perret (FR)**

(72) Inventeurs:
• **Queguiner, Claire**
**92260 Fontenay aux Roses (FR)**
• **Druesne, Anne**
**91440 Bures sur Yvette (FR)**
• **Alvarez Fernandez, Cristina**
**08290 Barcelona (ES)**
• **Pierson, Anne**
**91680 Bruyères le Chatel (FR)**
• **Colin, Cyril**
**91640 Janvry (FR)**

(74) Mandataire: **Boulinguiez, Didier**
**Cabinet Plasseraud**
**52 rue de la Victoire**
**75440 Paris Cedex 09 (FR)**

(56) Documents cités:
EP-A- 1 016 709        WO-A-01/32836
WO-A-01/32905        WO-A-20/04060073

• MASUDA O ET AL: "ANTIHYPERTENSIVE PEPTIDES ARE PRESENT IN AORTA AFTER ORAL ADMINISTRATION OF SOUR MILK CONTAINING THESE PEPTIDES TO SPONTANEOUSLY HYPERTENSIVE RATS" JOURNAL OF NUTRITION, WISTAR INSTITUTE OF ANATOMY AND BIOLOGY, PHILADELPHIA, PA,, US, vol. 126, no. 12, 1996, pages 3063-3068, XP002920598 ISSN: 0022-3166
• YMAMOTO N ET AL: "Antihypertensive effects of different kinds of fermented milk in spontaneously hypertensive rats" BIOSCIENCE BIOTECHNOLOGY BIOCHEMISTRY, JAPAN SOC. FOR BIOSCIENCE, BIOTECHNOLOGY AND AGROCHEM, TOKYO, JP, vol. 58, no. 4, 1994, pages 776-778, XP002095348 ISSN: 0916-8451

**Description**

La présente invention concerne de nouvelles souches de *Lactobacillus helveticus*, ainsi que leurs applications dans le domaine agro-alimentaire. Plus particulièrement, la présente invention propose des souches de *Lactobacillus helveticus* possédant de fortes capacités de productions de peptides anti-hypertension.

L'hypertension touche une part importante de la population. L'utilisation du peptide VPP (Val Pro Pro) et de peptides contenant la séquence VPP comme agents capables de réduire la pression artérielle par inhibition de l'enzyme de conversion de l'angiotensine (ACE) a été décrite dans EP 0 583 074. Un effet similaire du tripeptide IPP (Ile Pro Pro) a été également décrit dans la littérature. Ces peptides inhibent l'ACE en bloquant son site actif et l'empêchent ainsi de rendre active l'angiotensine.

Il est connu que les deux séquences VPP et IPP sont présentes dans la caséine Béta bovine et que l'hydrolyse adéquate de cette caséine (ou plus généralement du lait qui la contient) permet d'obtenir lesdits tripeptides. De nombreuses études chez l'animal et chez l'homme ont montré que l'ingestion journalière de quelques milligrammes de ces tripeptides permet de réduire efficacement la pression artérielle, en particulier chez les sujets hypertendus, réduisant d'autant le risque d'accident cardio-vasculaire.

Des peptides issus d'un lait fermenté par des *Lactobacillus helveticus* ont montré in vivo leur effet inhibiteur de l'ACE (ACEI) et ces peptides ont pu être retrouvés au niveau de l'aorte des rats ayant participé à l'étude (Masuda O. et al., 1996. J. Nutr. 126, 3063-8). D'autres études ont aussi montré plus spécifiquement une diminution de la pression artérielle chez des rats hypertendus suite à l'ingestion de peptides issus d'un lait fermenté par des *L. helveticus* (Yamamoto N. et al., 1994. Biosci. Biotech. Biochem. 58, 776-8).

Chez l'Homme, le même lait fermenté a aussi permis de diminuer la pression artérielle systolique (Hata . et al. ; 1996. Am. J. Clin. Nutr., 64, 767-71). Une étude plus récente confirme que les peptides présents dans le produit AMEAL S ® commercialisé par la société CALPIS réduisent de manière significative la pression artérielle chez des sujets ayant une pression artérielle supérieure à la normale (Mizuno et al., 2005. British Journal of Nutrition ; vol 94, issue 1, 84-91).

Dans une autre étude récente (Jauhiainen et al. ; 2005. Am. J. of Hypertension, 18 : 1600-1605) est émis l'hypothèse selon laquelle le mécanisme d'action ci-dessus cité (inhibition de l'ACE) pour expliquer l'effet antihypertenseur d'un lait fermenté par un *L. helveticus* pourrait cependant ne pas être le mécanisme d'action déterminant chez l'homme.

Une grande majorité des *Lactobacillus helveticus* est capable de produire les tripeptides IPP et VPP par fermentation de lait, mais les quantités produites peuvent être variables. EP 1 016 709 décrit un moyen de produire les tripeptides VPP et IPP par fermentation de lait à l'aide de souches de bactéries lactiques spécifiques appartenant à l'espèce *Lactobacillus helveticus*.

Toutefois, il est utile de pouvoir disposer de souches possédant des capacités à produire des quantités de tripeptides VPP et/ou IPP les plus élevées possibles.

La présente invention propose des souches possédant de telles capacités. En particulier, la présente invention propose des souches de *Lactobacillus helveticus* permettant la préparation de produits alimentaires possédant de très fortes teneurs en IPP et/ou VPP.

Par souche, on entend, au sens de la présente invention, toute culture, généralement pure, d'un micro-organisme, obtenue à partir d'une seule cellule ou d'une colonie isolée.

Par variant ou mutant d'une souche X, on entend désigner, au sens de la présente invention, toute souche obtenue à partir d'une souche de référence X. Dans le présent contexte, on utilisera plus particulièrement les termes « variant » pour désigner une souche obtenue principalement par mutation et sélection à partir d'une souche de référence X et « mutant » pour désigner plus spécifiquement une souche obtenue par des techniques de mutagénèse aléatoire ou dirigée (par exemple transformation génétique à l'aide de vecteurs), appliquées à la souche X.

Les mutants ou variants des souches selon la présente invention seront bien entendus concernés par la protection conférée par ce brevet à partir du moment où ils conservent les aspects essentiels de l'invention, notamment la capacité à produire de grandes quantités de IPP et/ou VPP.

Par souche à « phénotype fructose moins », on entend toute souche n'ayant pas la capacité à métaboliser le fructose.

Par milieu laitier, on entend tout milieu contenant des protéines de lait, par exemple un lait bovin standardisé à 4% en protéines avec de la poudre de lait bovin (écrémé ou non) ou avec du lait concentré.

Par produit laitier, au sens de la présente invention, on entend, en plus du lait, tout produit dérivé du lait, tel la crème, la crème glacée, le beurre, le fromage, le yogourt, le lait fermenté; les produits secondaires, comme le lactosérum, la caséine ainsi que divers aliments préparés contenant comme ingrédient principal du lait ou des constituants du lait. Parmi les produits laitiers, les produits laitiers fermentés comprennent entre autres les yaourts, les laits fermentés, les fromages blancs, les kéfirs, les fromages, les produits laitiers probiotiques et plus généralement tout produit laitier qui a subi au moins une étape de fermentation. Ledit lait est généralement du lait de vache, mais peut également être du lait d'autres mammifères, comme la chèvre, la brebis, la jument, la chamelle, la bufflonne.

Par produit alimentaire, au sens de la présente invention, on entend tout produit destiné à la nutrition humaine ou animale. En particulier, les produits alimentaires comprennent des produits destinés à l'alimentation des nourrissons, des enfants, des adolescents, et des adultes. Les produits alimentaires selon l'invention peuvent contenir en tout ou partie, au moins un produit laitier fermenté selon l'invention. Les produits alimentaires selon l'invention peuvent également contenir d'autres ingrédients habituellement utilisés dans l'industrie agroalimentaire, tels que des additifs, des conservateurs, des fruits ou extraits de fruits, des arômes, des colorants, des agents de texture, des céréales, des morceaux de chocolat, etc.

Par ferment, on entend, au sens de la présente invention, toute composition contenant au moins une souche vivante de micro-organisme susceptible de fermenter un milieu donné. Parmi les ferments, les ferments lactiques sont des compositions contenant au moins une souche vivante de micro-organisme susceptible de fermenter un milieu laitier.

Selon un mode de réalisation, l'invention concerne la souche de *Lactobacillus helveticus* I-3431 déposée le 25.05.05 auprès de la CNCM (Collection Nationale de Cultures de Micro-organismes), 28 rue du Docteur Roux, 75724 Paris, France, en accord avec les dispositions du Traité de Budapest.

Selon un mode de réalisation, la présente invention concerne les variants et les mutants de la souche de *Lactobacillus helveticus* I-3431 déposée le 25.05.05 auprès de la CNCM (Collection Nationale de Cultures de Micro-organismes), 28 rue du Docteur Roux, 75724 Paris, France, en accord avec les dispositions du Traité de Budapest capables, par fermentation à une température entre 30 et 45°C, plus préférentiellement entre 32 et 43°C et encore plus préférentiellement à 37°C d'un milieu laitier avec une teneur totale en protéines supérieure ou égale à 2% (p/p), préférentiellement entre 2 et 10% (p/p), plus préférentiellement entre 2,5 et 6% et encore plus préférentiellement égale à 4%, de produire les tripeptides de séquence IPP et/ou VPP en de fortes concentrations.

Un moyen d'exprimer la concentration en tripeptides de manière simple est de l'exprimer en concentration équivalent VPP [VPPeq].

On l'exprime en mg/kg : $[VPPeq] = [VPP] + (9/5 \times [IPP])$

Ainsi, selon un mode de réalisation, les souches de *Lactobacillus helveticus* selon l'invention sont avantageusement capables, par fermentation de produire les tripeptides de séquence IPP et/ou VPP en une quantité d'au moins 65 mg, préférentiellement au moins 70 mg, préférentiellement au moins 75 mg, préférentiellement au moins 80 mg, préférentiellement au moins 85 mg de VPPeq par kg de produit fermenté.

De telles quantités de VPP et/ou IPP sont généralement obtenues par fermentation par une souche selon l'invention à une température entre 30 et 45°C, préférentiellement entre 31 et 44°C, préférentiellement entre 32 et 43°C, préférentiellement entre 33 et 42°C, préférentiellement entre 34 et 41°C, préférentiellement entre 35 et 40°C, et plus préférentiellement à 37°C, d'un milieu laitier avec une teneur totale en protéines supérieure ou égale à 2% (p/p), préférentiellement entre 2 et 10% (p/p), plus préférentiellement entre 2,5 et 6% (p/p) et encore plus préférentiellement égale à 4% (p/p).

Selon un mode de réalisation, les souches de *Lactobacillus helveticus* selon l'invention possèdent en outre un phénotype fructose moins. En effet, le fructose a un fort pouvoir sucrant, et est un ingrédient utile dans les produits alimentaires. Ainsi, si le fructose ne peut être dégradé par la souche de *Lactobacillus helveticus*, le produit alimentaire conserve d'excellentes propriétés organoleptiques et le pH final sera avantageusement mieux contrôlé dans le cas où le produit alimentaire contient une préparation de fruit(s), notamment des morceaux et/ou du jus de fruit(s).

De manière avantageuse, selon un mode de réalisation, ledit produit alimentaire contient au moins $10^6$, préférentiellement au moins $10^7$, préférentiellement au moins $10^8$ UFC/mL de bactéries *Lactobacillus helveticus* vivantes. Ainsi, le produit alimentaire selon l'invention contient une importante biomasse.

Alternativement, le produit alimentaire selon l'invention peut être thermisé, de sorte à stabiliser son pH. Un tel produit alimentaire peut également être utilisé ultérieurement comme ingrédient dans un autre produit alimentaire.

Selon un mode de réalisation, ledit produit alimentaire contient au moins 25 mg, préférentiellement au moins 30 mg, préférentiellement au moins 35 mg, préférentiellement au moins 40 mg, préférentiellement au moins 45 mg, préférentiellement au moins 50 mg, préférentiellement au moins 55 mg, préférentiellement au moins 60 mg, préférentiellement au moins 65 mg, préférentiellement au moins 70 mg, préférentiellement au moins 75 mg, préférentiellement au moins 80 mg, préférentiellement au moins 85 mg de VPPeq par kg de produit alimentaire.

Selon un mode de réalisation, le produit alimentaire selon l'invention comprend en outre des préparations de fruit(s), notamment des morceaux et/ou du jus de fruit(s).

Avantageusement selon un mode de réalisation, le produit alimentaire selon l'invention possède des propriétés anti-hypertensives.

Selon un autre aspect, la présente invention concerne un procédé de préparation d'un produit alimentaire.

Selon un mode de réalisation, ledit procédé comprend les étapes suivantes :

■ sélectionner une matière première contenant des protéines de lait, notamment des protéines contenant les séquences peptidiques IPP et/ou VPP;

■ sélectionner au moins une souche *Lactobacillus helveticus* selon l'invention,

■ ensemencer ladite matière première avec ladite souche,

■ fermenter ladite matière première pendant 12-30 heures à 30-45°C,

■ optionnellement, thermiser le produit fermenté obtenu.

Selon un mode de réalisation, le procédé selon l'invention peut dépourvu de toute étape de thermisation après fermentation. Ceci permet de conserver des bactéries vivantes dans le produit alimentaire (pro-biotique).

Selon un autre mode de réalisation, ledit procédé peut comprendre une étape de thermisation.

L'invention sera davantage décrite à l'aide des exemples suivants, qui sont non-limitatifs.

## EXEMPLES DE REALISATION

### EXEMPLE 1 : Caractérisation d'une souche selon l'invention

La souche I-3431 a fait l'objet de dépôts auprès de la CNCM (Collection Nationale de Cultures de Micro-organismes), 28 rue du Docteur Roux, 75724 Paris, France, en accord avec les dispositions du Traité de Budapest le 25.05.05.

Cette souche a un phénotype fructose moins.

### EXEMPLE 2 : Mesure des propriétés d'acidification

Les propriétés d'acidification sont évaluées comme suit :

Un milieu contenant 120g de poudre de lait écrémé (PLE) dans 930ml d'eau est pasteurisé 10min à 95°C. Ce milieu est ensemencé à 0.5%, puis soumis à fermentation à 37°C. Le pH est alors suivi en fonction du temps.

Un prélèvement de milieu fermenté effectué à 30h de fermentation sera utilisé pour les mesures de production des tripeptides IPP et VPP ainsi que pour les mesures d'activité ACEI dont les résultats seront donnés ci-dessous.

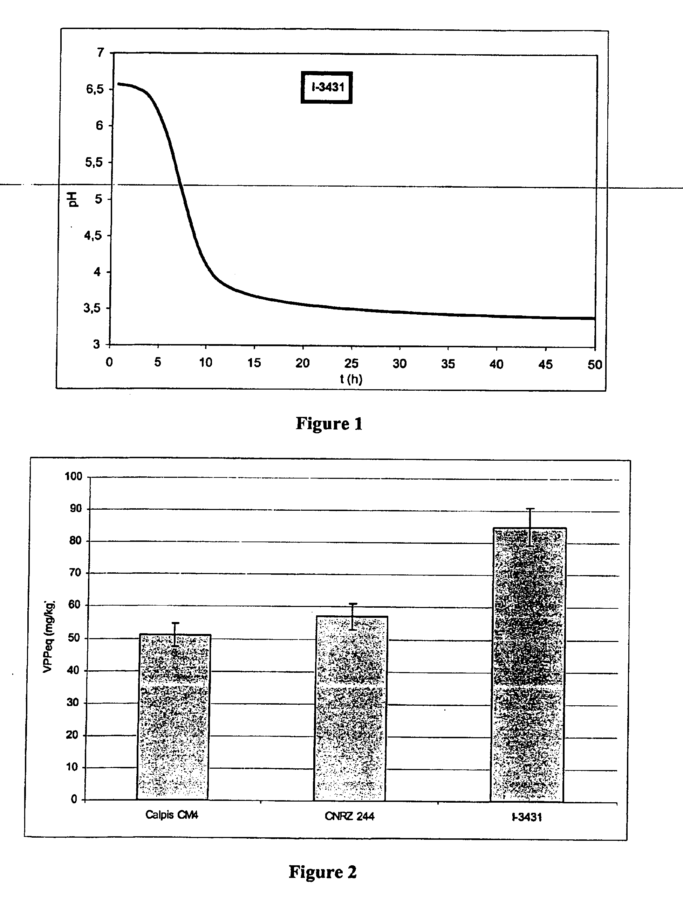

Les résultats présentés sur la figure 1 montrent les propriétés d'acidification d'une souche selon l'invention.

### EXEMPLE 3 : Mesure de la production des tripeptides IPP et VPP

Matériel et méthode

Un échantillon de milieu laitier (prélèvement à 30 heures de fermentation), tel que décrit précédemment dans l'exemple 2 est utilisé pour faire cette mesure.

L'analyse du contenu en peptides, notamment la teneur en tripeptides IPP et VPP, est conduite avec une méthode de chromatographie liquide CLHP couplée à un détecteur de type MS/MS comme décrit ci-après :

- Du fait des interférences inhérentes à l'analyse d'échantillons complexes, l'usage d'étalons internes deutérés ajoutés en quantité connue et maîtrisée au moment de la préparation de l'échantillon est fortement conseillée.

- La préparation de l'échantillon se fait par dilution du milieu fermenté dans un mélange d'eau, de méthanol et d'acide trifluoroacétique (50/50/0.1%), contenant 25 ppm d'étalon interne VPP deutéré (noté par la suite VPPd, de formule H-Val [D8]-Pro-Pro-OH, MM = 319,45 g/mol, disponible auprès de la société Bachem Chemicals, France) et 10 ppm d'étalon interne IPP deutéré (noté par la suite IPPd, de formule H-Ile [D10 N15]-Pro-Pro-OH, MM = 336,2 g/mol, disponible auprès de la société NEOMPS, Groupe SNPE, 7 rue de Boulogne, 67100 Strasbourg, France) dans un rapport de 1 à 3 (par exemple, pesée précise dans un eppendorf de 600 mg d'échantillon dans 1200 mg de mélange eau-méthanol-TFA contenant les étalons internes).

- Cet échantillon dilué est ensuite centrifugé à 14000 g pendant 15 minutes. Le surnageant clair obtenu, contenant les peptides générés pendant la fermentation, est ensuite dilué précisément au 1/50ème dans un mélange d'eau-méthanol (50/50, v/v) contenant 0,1% d'acide trifluoroacétique.

- La solution diluée ainsi obtenue est ensuite analysée dans un système chromatographique CLHP de type Agilent 1100 (société Agilent Technologies France, 1 rue Galvani 91745 Massy cedex, France), équipé d'une colonne adaptée à l'analyse des peptides, de type Waters Biosuite® (3 mm 2.1 x 150 mm, C18 PA-A, WAT186002427, Waters France, 5, Rue Jacques Monod, 78280 Guyancourt) à la température de 50°C, débit de 0.25 ml/min. Les peptides sont élués de façon classique avec un gradient croissant de solvant B (Acétonitrile + 0.100% d'acide formique) dans le solvant A (Eau + 0.106% acide formique), sur une durée de 35 min à 2 heures en fonction de la résolution chromatographique souhaitée.

La méthode adaptée au dosage des peptides IPP et VPP utilise le gradient suivant :

| Temps | % Tampon A | % Tampon B |
|-------|-----------|-----------|
| 0 | 100 | 0 |
| 3 | 96 | 4 |
| 6 | 89 | 11 |
| 15 | 60 | 40 |
| 18 | 0 | 100 |
| 21 | 100 | 0 |
| 24 | 0 | 100 |
| 27,5 | 100 | 0 |
| 35 | 100 | 0 |

- La détection se fait à l'aide d'un détecteur spécifique de type MS/MS, par exemple avec un appareil à trappe ionique comme l'Esquire3000+ (Bruker Daltonique, rue de l'Industrie, 67166 Wissembourg Cedex), paramétré en ionisation electrospray en mode positif, soit pour l'analyse globale du contenu peptidique (mode MS-MS), soit pour la quantification précise et spécifique d'un peptide à partir de ses fragments caractéristiques (mode MRM). Dans le cas du dosage des peptides IPP et VPP, mais aussi des étalons internes IPPd et VPPd, ces peptides sont isolés à partir de leur masse spécifique (ions monochargés 312,2 Da pour VPP ; 326,2 Da pour IPP ; 320,2 Da pour VPPd ; 337,3 Da pour IPPd) et quantifiés à partir de l'intensité de leurs ions spécifiques après fragmentation (fragments >= 85 Da).

L'intégration des pics chromatographiques de chacun des peptides IPP, VPP et la comparaison aux surfaces de pic des étalons internes de concentrations connues IPPd et VPPd permet ensuite de calculer, par régression linéaire simple, la teneur initiale de l'échantillon en VPP et IPP (généralement exprimée en mg/kg ou ppm).

Résultats

|  | VPPeq (mg/kg de milieu fermenté) |
|-------|-----------|
| Calpis CM4 | 51 |
| CNRZ 244 | 57 |
| I-3431 | 85 |

La figure 2 montre une comparaison de la production en tripeptides IPP et VPP de la souche I-3431 par rapport à deux souches de l'art antérieur.

La souche CM4 de la société CALPIS est décrite dans le brevet EP 1 016 709 tandis que la souche CRNZ 244 est décrite dans la demande de brevet WO 2004/060073.

Il est clair sur cette figure que la souche I-3431 (souche selon l'invention) a une production de tripeptides IPP et VPP bien supérieure à ces deux précédentes souches dans les mêmes conditions.

**EXEMPLE 4 : Mesure de l'activité ACEI (Inhibition de l'Enzyme de Conversion de l'Angiotensine)**

Matériel et méthode

Un échantillon de milieu laitier (prélèvement à 30 heures de fermentation), tel que décrit précédemment dans l'exemple 2 est utilisé pour faire cette mesure.

La présente méthode a pour base la méthode de Cushman et Cheng (Cushman et al. Biochem. Pharmacol. 1971. 20 :1637), adaptée pour la rendre compatible avec des échantillons de type produits laitiers fermentés.

## 1. Préparation des réactifs et solutions

### 1.1 Tampon borate de sodium 0.1M pH8.3, additionné de 0.3M de NaCl

Peser 6.1843g d'H$_3$BO$_3$ (Carlo Erba ref:402 766). Dissoudre dans environ 800 ml d'eau déminéralisée, ajuster à pH 8,3 avec une solution de NaOH puis ajouter 12,0 g de NaCl (concentration finale 0,3 M) et compléter à 1 litre avec l'eau déminéralisée.

### 1.2 Préparation du substrat HHL : solution de Hip-His-Leu à 5mM dans tampon borate de sodium 0.1M pH8.3 avec 0.3M de NaCl

Peser exactement 42.95 mg de peptide HHL anhydre (N-Hippuryl-Histidyl-Leucine tetrahydrate PM :501.5g, Sigma-Aldrich ref:53285-250 mg) et dissoudre dans environ 15 mL de tampon borate de sodium 0.1M pH8.3 avec 0.3M de NaCl puis compléter à 20mL avec ce même tampon.

### 1.3 Préparation de la solution d'ACE à 0.1U/mL

L'enzyme de conversion de l'angiotensine sous forme de poudre lyophilisée (origine : poumons de lapin, Sigma-Aldrich ref A6778, 0.25 unités) est solubilisée dans 2.5mL de tampon borate de sodium 0,1M pH 8,3 pour obtenir une solution à 0,1 U/mL. La solution ainsi préparée doit être utilisée, stockée à 4°C, au maximum 2 semaines pour préserver une activité enzymatique suffisante.

## 2. Préparation des échantillons de lait fermenté

Il est nécessaire de conditionner l'échantillon à un pH compris entre 8.0 et 8.5 de façon à être proche du pH optimal de l'ACE. Le lait fermenté est tout d'abord centrifugé, le pH du surnageant est ensuite ajusté entre 8.0 et 8.5 (idéalement pH 8.3) puis ultrafiltré à l'aide d'unité de filtration Vivaspin avec un seuil de coupure de 10 000 Daltons (Vivascience, France) afin d'éliminer les éléments interférents (protéines entières, sels de calcium).
Le protocole complet est le suivant :

- Déposer environ 6mL de lait fermenté dans un tube falcon 15mL après avoir préalablement pesé le tube vide. Peser la quantité de lait fermenté déposée dans le tube.

- Centrifuger à 14 000g pendant 10min à 10°C.

- Récupérer le surnageant - Quantifier la proportion culot/surnageant ce qui permet si nécessaire de déterminer l'IC50 équivalente au produit d'origine et non à son seul surnageant.

- Prélever 2.0mL de surnageant dans un tube à essais et mesurer le pH.

- Ajouter le volume nécessaire de NaOH 2M afin d'obtenir un pH compris entre 8.0 et 8.5 (cible : 8.3) après ajout du tampon borate, puis agiter.

- Ajouter le volume nécessaire de tampon borate pour diluer le surnageant de départ au ½ en tenant compte du volume exact de NaOH ajouté (par exemple : prise d'essai de surnageant = 2mL + 250μL NaOH + 1.75mL tampon borate)

- Vérifier que le pH final est compris entre 8.0 et 8.5. Au-delà, recommencer en ajoutant plus ou moins de NaOH. Un précipité se forme : il est dû aux sels de calcium.

- Ultracentrifuger l'échantillon à 12 000 g pendant 15 min à 10°C à l'aide d'unités de filtration Vivaspin 10 000 Daltons (capacité 4-6mL) afin d'obtenir un échantillon limpide.

## 3. Mesure de l'inhibition de l'ACE par les échantillons de laits fermentés

Lors de chaque série analyse, des témoins contrôles (0 et 100% activité ACE) doivent être préparés. Pour chaque échantillon, deux essais indépendants et un blanc échantillon sont réalisés pour chaque dilution. Il faut en effet ajuster au plus près (en diluant) la quantité d'échantillon nécessaire pour diminuer de 50% l'activité de l'ACE.

Pour cela :
- Déposer 80μL de l'échantillon conditionné à pH8.3 dans le tube blanc et dans les tubes essais
- Déposer 80μL d'eau déminéralisée dans les tubes contrôle 0 et 100%
- Ajouter 200μl de la solution de substrat HHL à 5mM dans tous les tubes. Agiter.
- Placer les tubes dans un bain d'eau thermostaté à 37°C, laisser s'équilibrer en température
- Déclencher la réaction enzymatique par ajout dans chaque tube de 20μL de la solution d'ACE à 0.1U/mL, sauf pour les blancs échantillons et contrôle 0% pour lesquels il faut déposer 20μL de tampon borate pH 8.3.
- Laisser hydrolyser pendant 1h exactement à 37°C.
- Arrêter la réaction en ajoutant dans chaque tube 250μL de la solution d'HCl 1M. Tableau récapitulatif de la composition des différents milieux réactionnels :

|  | Prise d'essai | Ajout du Substrat | Déclenchement hydrolyse |
|---|---|---|---|
| **100% contrôle** | 80μL eau déminéralisée | 200μL HHL | 20μL ACE |
| **0% contrôle** | 80μL eau déminéralisée | 200μL HHL | 20μL tampon borate |
| **Echantillon (essai)** | 80μL échantillon | 200μL HHL | 20μL ACE |
| **Blanc échantillon** | 80μL échantillon | 200μL HHL | 20μL tampon borate |

La lecture se fait par extraction du substrat hydrolysé (acide hippurique) et sa quantification à l'aide d'un spectrophotomètre, avec le protocole suivant :

Ajouter dans chaque tube 1.7mL acétate d'éthyle, agiter.
Centrifuger à 2000g pendant 5 min à 10°C
Prélever 1mL exactement du surnageant dans un micro-tube eppendorf
Evaporer l'acétate d'éthyle à 120°C pendant 10min dans un bloc chauffant
Ajouter exactement 1mL d'eau déminéralisée puis agiter 10 sec pour reprendre l'acide hippurique.
Lire l'absorbance à 228 nm dans des cuves adaptées à la lecture dans l'UV

Expression des résultats :

Le pourcentage d'inhibition de l'ACE est calculé comme suit :

$$\frac{(\text{Abs 100\% ctl} - \text{Abs 0\% ctl}) - (\text{Abs éch} - \text{Abs blanc éch})}{(\text{Abs 100\% ctl} - \text{Abs 0\% ctl})} \times 100$$

Avec Abs = absorbance à 228 nm après extraction de l'acide hippurique
ctl = contrôle
éch = échantillon
Pour un lait fermenté, nous exprimons l'IC50 comme étant la quantité de surnageant de ce lait fermenté qui inhibe 50% de l'activité enzymatique de l'ACE dans le milieu réactionnel, soit en microlitres de surnageant de lait fermenté par millilitre de milieu réactionnel.

Résultats

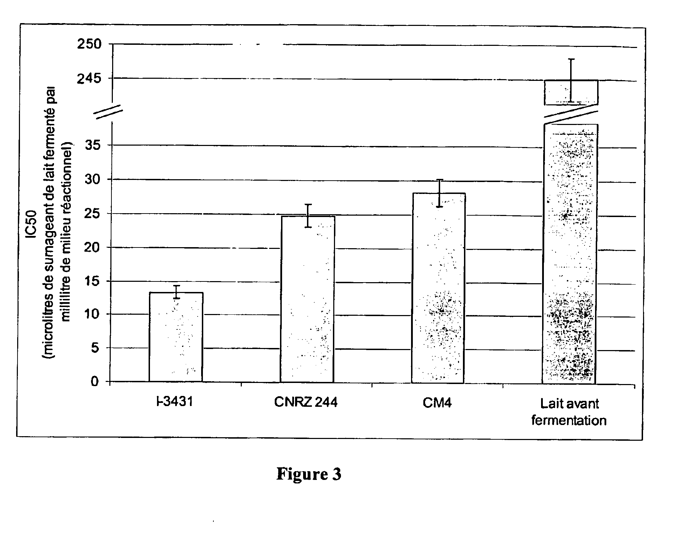

La figure 3 montre une comparaison de l'activité inhibitrice de l'enzyme de conversion de l'angiotensine des différentes souches selon la présente invention comparées à des souches de l'art antérieur.
En ordonnée est exprimée la concentration équivalente de lait fermenté par ml de milieu réactionnel nécessaire pour inhiber 50% de l'activité de l'enzyme de conversion de l'angiotensine (IC50). Plus cette concentration est élevée, moins la souche a donc de capacité à inhiber l'enzyme de conversion de l'angiotensine.

**EXEMPLE 5 : préparation d'un produit laitier (probiotique) selon l'invention**

Du lait de grand mélange, préalablement écrémé, pré-pasteurisé et refroidi à 4°C est standardisé en protéines (4.0%) avec de la poudre de lait écrémé. Le milieu laitier ainsi préparé est soumis à une pasteurisation (95°C-8 min).

Après refroidissement à 37°C le milieu laitier est ensemencé avec la souche I-3431 à raison de $10^7$ UFC/ mL et maintenu à 37°C pendant toute la durée de la fermentation. Lorsque le pH 3.5 est atteint, le caillé est soutiré de la cuve de fermentation pour subir un lissage (par passage au travers d'un filtre) et refroidi jusqu'à 10°C dans un échangeur à plaque. Le caillé lissé et refroidi est ensuite additionné d'une préparation de fruits (ayant un pH entre 4 et 4,1) à raison de 15% du produit fini, conditionné en flacons de 110 mL et stocké à 4°C pendant 28 jours. Le produit ainsi préparé contient des tripeptides de séquence IPP/VPP à raison de 70 mg/kg d'équivalent VPP. La teneur en peptides du produit est stable durant toute la durée de vie du produit.

**Revendications**

1. Souche de *Lactobacillus helveticus* I-3431 déposée le 25.05.05 auprès de la CNCM (Collection Nationale de Cultures de Micro-organismes), 28 rue du Docteur Roux, 75724 Paris, France, en accord avec les dispositions du Traité de Budapest,
   ses variants et ses mutants capables, par fermentation à une température entre 30 et 45°C, d'un milieu laitier avec une teneur totale en protéines supérieure ou égale à 2% (p/p), de produire les tripeptides de séquence IPP et/ou VPP en une quantité d'au moins 65 mg de VPPeq par kg de produit fermenté.

2. Souche de *Lactobacillus helveticus* selon la revendication 1, lesdits variants et mutants étant capables, par fermentation à une température entre 30 et 45°C, d'un milieu laitier avec une teneur totale en protéines supérieure ou égale à 2% (p/p), de produire les tripeptides de séquence IPP et/ou VPP en une quantité d'au moins 70 mg de VPPeq par kg de produit fermenté.

3. Souche de *Lactobacillus helveticus* selon la revendication 1, lesdits variants et mutants étant capables, par fermentation à une température entre 30 et 45°C, d'un milieu laitier avec une teneur totale en protéines supérieure ou égale à 2% (p/p), de produire les tripeptides de séquence IPP et/ou VPP en une quantité d'au moins 75 mg de VPPeq par kg de produit fermenté.

4. Souche de *Lactobacillus helveticus* selon la revendication 1, lesdits variants et mutants étant capables, par fermentation à une température entre 30 et 45°C, d'un milieu laitier avec une teneur totale en protéines supérieure ou égale à 2% (p/p), de produire les tripeptides de séquence IPP et/ou VPP en une quantité d'au moins 80 mg de VPPeq par kg de produit fermenté.

5. Souche de *Lactobacillus helveticus* selon la revendication 1, lesdits variants et mutants étant capables, par fermentation à une température entre 30 et 45°C, d'un milieu laitier avec une teneur totale en protéines supérieure ou égale à 2% (p/p), de produire les tripeptides de séquence IPP et/ou VPP en une quantité d'au moins 85 mg de VPPeq par kg de produit fermenté.

6. Souche de *Lactobacillus helveticus* selon l'une quelconque des revendications 1-5, qui possède en outre un phénotype fructose moins.

7. Utilisation d'une souche de *Lactobacillus helveticus* selon l'une quelconque des revendications 1-6, pour la préparation d'un produit alimentaire ou pharmaceutique, notamment d'un produit laitier fermenté.

8. Utilisation selon la revendication 7, telle que ledit produit alimentaire ou pharmaceutique a des propriétés anti-hypertensives.

9. Produit alimentaire, notamment produit laitier fermenté, comprenant au moins une souche de *Lactobacillus helveticus* selon l'une des revendications 1-6.

10. Produit alimentaire selon la revendication 9, contenant au moins $10^6$ UFC/mL de bactéries *Lactobacillus helveticus* vivantes.

11. Produit alimentaire selon la revendication 9, contenant au moins $10^7$ UFC/mL de bactéries *Lactobacillus helveticus* vivantes.

12. Produit alimentaire selon la revendication 9, contenant au moins $10^8$ UFC/mL de bactéries *Lactobacillus helveticus* vivantes.

**13.** Produit alimentaire selon la revendication 9, contenant au moins 25 mg de VPPeq par kg dudit produit alimentaire.

**14.** Produit alimentaire selon la revendication 9, contenant au moins 50 mg de VPPeq par kg dudit produit alimentaire.

**15.** Produit alimentaire selon la revendication 9, contenant au moins 75 mg de VPPeq par kg dudit produit alimentaire.

**16.** Produit alimentaire selon l'une quelconque des revendications 9-15, comprenant en outre des préparations de fruit (s), notamment des morceaux et/ou du jus de fruit(s).

**17.** Produit alimentaire selon l'une quelconque des revendications 9-16, qui possède des propriétés anti-hypertensives.

**18.** Procédé de préparation d'un produit alimentaire comprenant les étapes suivantes :

- ■ sélectionner une matière première contenant des protéines de lait, notamment des protéines contenant les séquences peptidiques IPP et/ou VPP;
- ■ sélectionner au moins une souche *Lactobacillus helveticus* selon l'une des revendications 1-6,
- ■ ensemencer ladite matière première avec ladite souche,
- ■ fermenter ladite matière première pendant 12-30 heures à 30-45°C,
- ■ optionnellement, thermiser le produit fermenté obtenu.

**Claims**

**1.** Strain of *Lactobacillus helveticus* I-3431 deposited at the CNCM (*Collection Nationale de Cultures de Micro-organismes* = National Collection of Cultures of Microorganisms), 28 rue du Docteur Roux, 75724 Paris, France, on 25.5.05, in accordance with the provisions of the Treaty of Budapest,
its variants and mutants capable of producing, through fermentation at a temperature between 30 and 45°C of a milk medium with a total proteins content of not less than 2% (p/p), tripeptides of the sequence IPP and/or VPP in a quantity of at least 65 mg of VPPeq per kg of fermented product.

**2.** Strain of *Lactobacillus helveticus* according to Claim 1, the said variants and mutants being capable of producing, through fermentation at a temperature between 30 and 45 °C of a milk medium with a total proteins content of not less than 2% (p/p), tripeptides of the sequence IPP and/or VPP in a quantity of at least 70 mg of VPPeq per kg of fermented product.

**3.** Strain of *Lactobacillus helveticus* according to Claim 1, the said variants and mutants being capable of producing, through fermentation at a temperature between 30 and 45 °C of a milk medium with a total proteins content of not less than 2% (p/p), tripeptides of the sequence IPP and/or VPP in a quantity of at least 75 mg of VPPeq per kg of fermented product.

**4.** Strain of *Lactobacillus helveticus* according to Claim 1, the said variants and mutants being capable of producing, through fermentation at a temperature between 30 and 45°C of a milk medium with a total proteins content of not less than 2% (p/p), tripeptides of the sequence IPP and/or VPP in a quantity of at least 80 mg of VPPeq per kg of fermented product.

**5.** Strain of *Lactobacillus helveticus* according to Claim 1, the said variants and mutants being capable of producing, through fermentation at a temperature between 30 and 45 °C of a milk medium with a total proteins content of not less than 2% (p/p), tripeptides of the sequence IPP and/or VPP in a quantity of at least 85 mg of VPPeq per kg of fermented product.

**6.** Strain of *Lactobacillus helveticus* according to any one of Claims 1-5 which also possesses a fructose minus phenotype.

**7.** Use of a strain of *Lactobacillus helveticus* according to any one of Claims 1-6 for the preparation of a food or pharmaceutical product, in particular a fermented milk product.

**8.** Use according to Claim 7 such that the said food or pharmaceutical product has anti-hypertensive properties.

9. Food product, in particular a fermented milk product, comprising at least one strain of *Lactobacillus helveticus* according to any one of Claims 1-6.

10. Food product according to Claim 9 containing at least $10^6$ UFC/mL of live *Lactobacillus helveticus* bacteria.

11. Food product according to Claim 9 containing at least $10^7$ UFC/mL of live *Lactobacillus helveticus* bacteria.

12. Food product according to Claim 9 containing at least $10^8$ UFC/mL of live *Lactobacillus helveticus* bacteria.

13. Food product according to Claim 9 containing at least 25 mg of VPPeq per kg of the said food product.

14. Food product according to Claim 9 containing at least 50 mg of VPPeq per kg of the said food product.

15. Food product according to Claim 9 containing at least 75 mg of VPPeq per kg of the said food product.

16. Food product according to any one of Claims 9-15, also comprising preparations of fruit(s), in particular fruit pieces and/or juice(s).

17. Food product according to any one of Claims 9-16 which possesses anti-hypertensive properties.

18. Process for the preparation of a food product comprising the steps of:

   • selecting a starting material containing milk proteins, in particular proteins containing the peptidic sequences IPP and/or VPP;
   • selecting at least one *Lactobacillus helveticus* strain according to any one of Claims 1-6,
   • inoculating the said starting material with the said strain,
   • fermenting the said starting material for 12-30 hours at 30-45°C,
   • optionally, thermizing the fermented product obtained.

**Patentansprüche**

1. Stamm von Lactobacillus helveticus 1-3431, der am 25.05.05 bei der CNCM (Collection Nationale de Cultures de Micro-organismes), 28 rue du Docteur Roux, 75724 Paris, Frankreich, in Übereinstimmung mit den Bestimmungen des Budapester Vertrags hinterlegt wurde, seine Varianten und seine Mutanten, die in der Lage sind, durch Fermentation bei einer Temperatur zwischen 30 und 45 °C eines Milchmediums mit einem Gesamtgehalt an Proteinen von größer oder gleich 2 Ges.-%, Tripeptide mit der Sequenz IPP und/oder VPP in einer Menge von mindestens 65 mg VPPeq je kg fermentiertem Produkt zu produzieren.

2. Stamm von Lactobacillus helveticus nach Anspruch 1, wobei seine Varianten und Mutanten in der Lage sind, durch Fermentation bei einer Temperatur zwischen 30 und 45°C eines Milchmediums mit einem Gesamtgehalt an Proteinen von größer oder gleich 2 Ges.-%, Tripeptide mit der Sequenz IPP und/oder VPP in einer Menge von mindestens 70 mg VPPeq je kg fermentiertem Produkt zu produzieren.

3. Stamm von Lactobacillus helveticus nach Anspruch 1, wobei seine Varianten und Mutanten in der Lage sind, durch Fermentation bei einer Temperatur zwischen 30 und 45°C eines Milchmediums mit einem Gesamtgehalt an Proteinen von größer oder gleich 2 Gew.-%, Tripeptide mit der Sequenz IPP und/oder VPP in einer Menge von mindestens 75 mg VPPeq je kg fermentiertem Produkt zu produzieren.

4. Stamm von Lactobacillus helveticus nach Anspruch 1, wobei seine Varianten und Mutanten in der Lage sind, durch Fermentation bei einer Temperatur zwischen 30 und 45 °C eines Milchmediums mit einem Gesamtgehalt an Proteinen von größer oder gleich 2 Gew.-%, Tripeptide mit der Sequenz IPP und/oder VPP in einer Menge von mindestens 80 mg VPPeq je kg fermentiertem Produkt zu produzieren.

5. Stamm von Lactobacillus helveticus nach Anspruch 1, wobei seine Varianten und Mutanten in der Lage sind, durch Fermentation bei einer Temperatur zwischen 30 und 45 °C eines Milchmediums mit einem Gesamtgehalt an Proteinen von größer oder gleich 2 Gew.-%, Tripeptide mit der Sequenz IPP und/oder VPP in einer Menge von mindestens 85 mg VPPeq je kg fermentiertem Produkt zu produzieren.

**6.** Stamm von Lactobacillus helveticus nach einem der Ansprüche 1 bis 5, der ferner einen fructosenegativen Phänotyp besitzt.

**7.** Verwendung eines Stamms von Lactobacillus helveticus nach einem der Ansprüche 1 bis 6 zur Herstellung eines Nahrungsmittels oder eines Arzneimittels, insbesondere eines fermentierten Milchprodukts.

**8.** Verwendung nach Anspruch 7, wobei das Nahrungsmittel oder Arzneimittel blutdrucksenkende Eigenschaften aufweist.

**9.** Nahrungsmittel, insbesondere fermentiertes Milchprodukt, das mindestens einen Stamm von Lactobacillus helveticus nach einem der Ansprüche 1 bis 6 umfasst.

**10.** Nahrungsmittel nach Anspruch 9, das mindestens $10^6$ CFU/ml lebende Lactobacillus-helveticus-Bakterien enthält.

**11.** Nahrungsmittel nach Anspruch 9, das mindestens $10^7$ CFU/ml lebende Lactobacillus-helveticus-Bakterien enthält.

**12.** Nahrungsmittel nach Anspruch 9, das mindestens $10^8$ CFU/ml lebende Lactobacillus-helveticus-Bakterien enthält.

**13.** Nahrungsmittel nach Anspruch 9, das mindestens 25 mg VPPeq je kg des Nahrungsmittels enthält.

**14.** Nahrungsmittel nach Anspruch 9, das mindestens 50 mg VPPeq je kg des Nahrungsmittels enthält.

**15.** Nahrungsmittel nach Anspruch 9, das mindestens 75 mg VPPeq je kg des Nahrungsmittels enthält.

**16.** Nahrungsmittel nach einem der Ansprüche 9 bis 15, das ferner Zubereitungen einer Frucht (von Früchten), insbesondere Stücke einer Frucht (von Früchten) und/oder Saft einer Frucht (von Früchten) enthält.

**17.** Nahrungsmittel nach einem der Ansprüche 9 bis 16, das blutdrucksenkende Eigenschaften besitzt.

**18.** Verfahren zur Herstellung eines Nahrungsmittels, das die folgenden Schritte umfasst:

■ Auswählen eines Grundstoffs, der Milchproteine enthält, insbesondere Proteine, die die Peptidsequenzen IPP und/oder VPP enthalten;
■ Auswählen mindestens eines Lactobacillus-helveticus-Stamms nach einem der Ansprüche 1 bis 6,
■ Animpfen des Grundstoffs mit dem Stamm,
■ Fermentieren des Grundstoffs während 12 bis 30 Stunden bei 30 bis 45 °C,
■ optional Thermisieren des erhaltenen fermentierten Produkts.

**Figure 1**

**Figure 2**

**Figure 3**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0583074 A
- EP 1016709 A
- WO 2004060073 A

**Littérature non-brevet citée dans la description**

- **MASUDA O. et al.** *J. Nutr.,* 1996, vol. 126, 3063-8
- **YAMAMOTO N. et al.** *Biosci. Biotech. Biochem.,* 1994, vol. 58, 776-8
- **HATA et al.** *Am. J. Clin. Nutr.,* 1996, vol. 64, 767-71
- **MIZUNO et al.** *British Journal of Nutrition,* 2005, vol. 94 (1), 84-91
- **JAUHIAINEN et al.** *Am. J. of Hypertension,* 2005, vol. 18, 1600-1605
- **CUSHMAN et al.** *Biochem. Pharmacol.,* 1971, vol. 20, 1637